# EUROPÄISCHE PATENTSCHRIFT

(11) **EP 2 964 274 B1**
(45) Veröffentlichungstag und Bekanntmachung des Hinweises auf die Patenterteilung: **05.05.2021**
(21) Anmeldenummer: 14715219.3
(22) Anmeldetag: 04.03.2014
(51) Int. Cl.: A61L 2/14

(54) **VERFAHREN UND VORRICHTUNG ZUR PLASMABEHANDLUNG VON HOHLKÖRPERN**
METHOD AND DEVICE FOR PLASMA-TREATING HOLLOW BODIES
PROCÉDÉ ET DISPOSITIF SERVANT AU TRAITEMENT PAR PLASMA DE CORPS CREUX

(30) Priorität: 04.03.2013 DE 102013203648
(43) Veröffentlichungstag der Anmeldung: 13.01.2016
(73) Patentinhaber: Leibniz-Institut für Plasmaforschung und Technologie e.V., 17489 Greifswald (DE)
(72) Erfinder: EHLBECK, Joerg, 17498 Hinrichshagen (DE); STIEBER, Manfred, 17489 Greifswald (DE); WELTMANN, Klaus-Dieter, 18609 Binz (DE)
(74) Vertreter: Schulz Junghans Patentanwälte PartGmbB
(86) Internationale Anmeldenummer: PCT/EP2014/054166
(87) Internationale Veröffentlichungsnummer: WO 2014/135531

(56) Entgegenhaltungen:
- EP-A1- 2 008 670
- DE-A1- 19 807 742
- DE-A1- 19 916 479
- US-A- 5 897 831

## Beschreibung

### Beschreibung der Erfindung

Die vorliegende Erfindung betrifft eine Vorrichtung zur Plasmabehandlung von Hohlkörpern, wie beispielsweise Flaschen, Vials, Syringen, Verschluss-Kappen etc., insbesondere mit dem Ziel der Sterilisation dieser Hohlkörper, und ist für verschiedene Entladungstypen wie DBE (Dielektrisch Behinderte Entladung) und Entladungen mit HF-und Mikrowellenanregung geeignet. Kernstück der Erfindung ist ein aus drei, vorzugsweise konzentrisch angeordneten Rohren (Hülsen), bestehendes System, wobei die innere Hülse als Innenleiter bzw. Innenelektrode dient, die mittlere den - partiell auf der Grundplatte abgedichteten - Außenleiter bzw. die Außenelektrode darstellt und die Außenhülse über eine Dichtung am Aufsatzende verfügt. Die Vorrichtung ermöglicht es, ein Druckgefälle zwischen Innenraum und Außenraum des Behandlungsgutes einzustellen, was für die Prozessführung und Plasmaausbildung vorteilhaft genutzt werden kann und die Grundlage für ein optimiertes In-Line-Verfahren der Plasmabehandlung von Hohlkörpern bildet.

### Stand der Technik

Insbesondere in der pharmazeutischen Industrie besteht ein hoher Bedarf an Hochratensterilisationsverfahren für überwiegend kleine Hohlkörper im Bereich von 2 bis 100 ml. Typische Beispiele hierfür sind Vials und Syringes.

Es besteht ein zunehmender Wunsch, die klassischen thermischen Verfahren, obwohl als sehr sichere Verfahren bewährt, durch moderne Methoden abzulösen. Die erwarteten Hauptvorteile sind kürzere Prozesszeiten, insbesondere die Vermeidung von Abkühlpuffern, und die damit einhergehende kompaktere Bauform, bzw. ein höherer Durchsatz bei gleichem Bauvolumen.

Verfahren der Gassterilisation, wie z.B. die Verwendung von Ethylenoxid, Formaldehyd oder Wasserstoffperoxid, sind problematisch in der Applikation und bergen das Risiko einer negativen Wechselwirkung mit dem abzufüllenden pharmazeutischen Präparat. Auch plasmabasierte indirekte Verfahren wie Ozonisierung oder das P PA-Verfahren, die ein Havarie-Risiko minimieren, Unterliegen diesem Wechselwirkungsrisiko.

Direkte Plasmaverfahren bei Atmosphärendruck, wie z.B. die Anwendung HFangeregter Plasmajets, scheitern bei Edelgasbetrieb an den durch den hohen Gasverbrauch verursachten Betriebskosten. Bei Übergang zu Luftbetrieb, sofern technisch umsetzbar, sinkt einerseits die Lebensdauer und steigt die Gastemperatur, so dass eine Eignung für thermolabile Materialien fraglich ist.

Ein weiteres Beispiel für kalte Plasmen bei Atmosphärendruck ist die DBE, die aber aufgrund der Formgebung der Hohlkörper große Schlagweiten realisieren müsste. Die hierzu erforderlichen Hochspannungen erschweren eine industrielle Applikation.

Niederdruckreaktoren verursachen durch aufwändige und - insbesondere bei Batchbetrieb - große Behältnisse mit hohen Totvolumina hohe Investitionskosten. Die bei hohen Durchsatz-Raten erforderlichen Pumpraten sind ein weiterer Kostenfaktor für Investition und Betrieb.

Zum Stand der Technik gehören auch die Druckschriften DE 199 16 479 A1 und DE 10 2008 034 111 A1. In der DE 199 16 479 A1 wird eine Vorrichtung zum Sterilisieren von Behältern mittels Plasma bei Niederdruck offenbart. Bei dem beschriebenen Plasma handelt es sich um ein Hochfrequenzplasma. Ein Atmosphärendruckplasma oder eine dielektrisch behinderte Entladungen wird darin nicht beschrieben. Ein Dichtsystem, basierend auf einer vorzugsweise konzentrisch angeordneten Doppelkammer, ist ebenfalls nicht Gegenstand von DE 199 16 479 A1.

Die Druckschrift DE 10 2008 034 111 A1 beschreibt eine Vorrichtung zum Sterilisieren und/oder Entkeimen von Werkstücken aus Kunststoff, vorzugsweise Verschlusskappen unter Atmosphärendruck mittels eines Plasmas. Somit wird auch in dieser Schrift nicht auf eine spezielle Art eines Doppelkammersystems zur Generierung einer hohen Prozessgasreinheit eingegangen.

Die EP 2 008 670 A1 offenbart einen Sterilisator zur effizienten Sterilisation eines Gegenstandes, wie z.B. eines Behälters, unter Verwendung von Sauerstoff und Plasma, sowie ein Sterilisationsverfahren. In der Sterilisationseinrichtung wird dabei der Gegenstand mit Plasma kontaktiert. Dazu werden ein nicht-leitendes Gaskanalrohr zur Zuführung des in Plasma umzuwandelnden Gases und eine leitfähige Antennenröhre zu Verfügung gestellt, wobei die Antennenröhre einen Schlitz mit einer bestimmten Länge entlang der Röhrenachsenrichtung des Gaskanalrohrs aufweist, und das Antennenröhre mit Mikrowellen bestrahlt wird, um das Gas in dem Gaskanalrohr in Plasma umzuwandeln.

Keine der bekannten technischen Lösungen offenbart eine effiziente kostensparende Lösung, in Hochratenprozessen definierte Prozessgasatmosphären zu generieren. Diese sind essentiell zum Erzielen stabiler Prozessbedingungen. Kleinste Verunreinigungen können -insbesondere bei Sterilisationsprozessen - zu einem vollständigen Prozessversagen führen.

Die in den genannten Schriften aufgeführten Methoden der Plasmagenerierung sind als solche schon seit Jahren Stand der Technik.

### Aufgabe der Erfindung

Der Erfindung liegt die Aufgabe zugrunde, die Nachteile der im Stand der Technik beschriebenen Lösungen zu beseitigen.

### Lösung der Aufgabe

Die Aufgabe wurde durch eine Vorrichtung zur Plasma-Behandlung von Hohlkörpern und eines entsprechenden Verfahrens gemäß den Merkmalen der Patentansprüche gelöst. Erfindungsgemäß ist eine Vorrichtung vorgesehen, die eine Grundplatte und ein elektrisch leitendes Innenrohr umfasst. Die erfindungsgemäße Vorrichtung ist dadurch gekennzeichnet, dass die Vorrichtung weiterhin zwei Außenrohre aufweist, nämlich ein inneres Außenrohr und ein äußeres Außenrohr, von denen mindestens eines elektrisch leitfähig ist, und das Innenrohr und das innere Außenrohr voneinander galvanisch isoliert sind und miteinander gasdicht verbunden sind, so dass sie eine Vorkammer zwischen den zwei Außenrohren und einen Behandlungsraum zwischen dem inneren Außenrohr und dem Innenrohr bilden, wobei das äußere Außenrohr mit der Grundplatte über eine Dichtung verbindbar oder verbunden ist, und das innere Außenrohr über eine einstellbare Kontaktdichtung mit der Grundplatte verbindbar oder verbunden ist.

Ein wesentliches Merkmal der Erfindung ist die erstmalige Kombination von Plasmaprozess und Dichtsystem. Das wird gemäß des Hauptanspruchs so realisiert, dass die Außenrohre 4, 6, von denen mindestens eines elektrisch leitfähig ist, miteinander gasdicht, aber galvanisch isoliert, verbunden sind, so dass sie eine Vorkammer zwischen den zwei Außenrohren 4, 6 und einen Behandlungsraum zwischen dem Außenrohr 6 und dem Innenrohr 1 bilden. Damit entsteht ein Doppelkammersystem.

### Darstellung der Erfindung

Die vorliegende Erfindung ist für verschiedene Entladungstypen wie DBD, HF-und Mikrowellenanregung geeignet. Sie beschreibt ein System aus drei vorzugsweise konzentrisch angeordneten Rohre (Hülsen). Die innere dient als Innenleiter bzw. Innenelektrode, die mittlere stellt den Außenleiter oder die Außenelektrode dar. Sie dichtet partiell auf der metallischen Transportplatte. Die Außenhülse verfügt über eine Dichtung am Aufsatzende. Ziel ist es, die Gaszuführung mit leichtem Überdruck durch den Spalt zwischen Außen- und Mittelhülse zu führen. Dadurch führen Leckagen im Dichtsystem zu keiner Verunreinigung der Prozessgase. Gas-Strom und Arbeitsdruck können über die Dichtigkeit der Kontaktfläche der Mittelhülse zur Transportplatte eingestellt werden. Die freie Wahl des Arbeitsgases sowie die Reduzierung des Arbeitsdruckes erlauben es, auch mittels DBE Vials mit ihrer typischen Formgebung mit moderaten Spannungen zu betreiben. Eine grobe Zentrierung des Vials auf die Vorrichtung kann durch den Absenkprozess erfolgen. Die Feinjustierung kann im abgesenkten Modus durch Injektion eines nicht zwingend sterilen tangentialen Gas-Stromes erfolgen. Die Einzelvorrichtung kann zu entsprechenden Balken kombiniert werden. Diese können durch beidseitige Transportketten parallel mit dem Transport der Vials mitgeführt werden. Mittels Werkzeugwechsel kann die Anlage an stark unterschiedliche Größen ange-passt werden. Dazu kann auf der Seite, die der Behandlungsstelle abgewandt ist, eine Werkzeugwechselstation und ein Werkzeugmagazin installiert werden, um so einen vollautomatischen Werkzeugwechsel durchzuführen.

Somit werden folgende Hauptprobleme durch die Erfindung gelöst:
1. Keine Verwendung von aufwendigen Vakuumreaktoren
2. Minimierung des Totvolumens, daraus folgt eine Reduzierung der Pumpenleistung
3. Durch die spezielle Gasführung wurde das Dichtungsproblem gelöst
4. Durch Anwendung im Nieder- bis Mitteldruck kann mit einer großen Bandbreite von Prozessgasen gearbeitet werden (z.B. Luft. Edelgase, Formiergas, Zusatz von Feuchtigkeit ...)
5. Formentoleranz ist gegeben

Gegenüber häufig eingesetzten Batchverfahren hat man die Vorteile eines In-line-Verfahrens. Hinzu kommt der Vorteil, dass das zu evakuierende Volumen in der Vorrichtung minimiert wurde, welches Einerseits zu geringeren Kosten hinsichtlich des Evakuierungsprozesses führt und insgesamt die Prozesszeiten minimiert. Ferner werden auch die Mengen an erforderlichen Prozessgasen, insbesondere Precursoren beim Beschichten, reduziert.

Der Vorteil gegenüber klassischen In-line-Plasmaprozessen ist hier vor allem im reduzierten Aufwand für das Dichtsystem zu sehen. Ein weiterer wichtiger Vorteil ist die erhöhte Prozesssicherheit im Falle eines Versagens des Dichtsystems.

### Ausführungsbeispiele

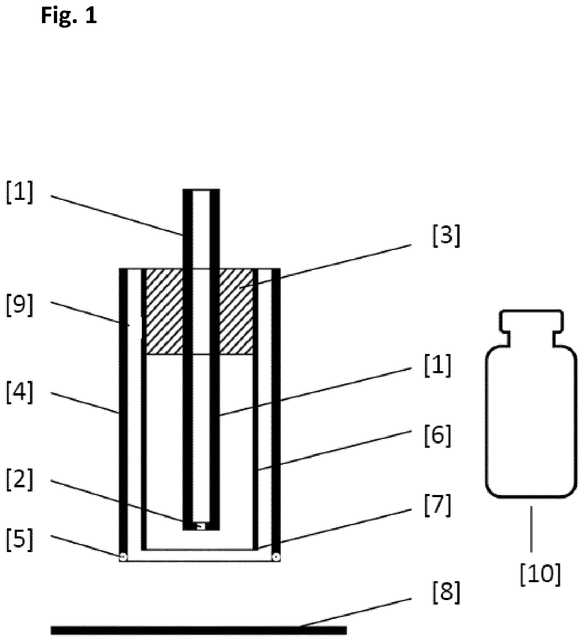

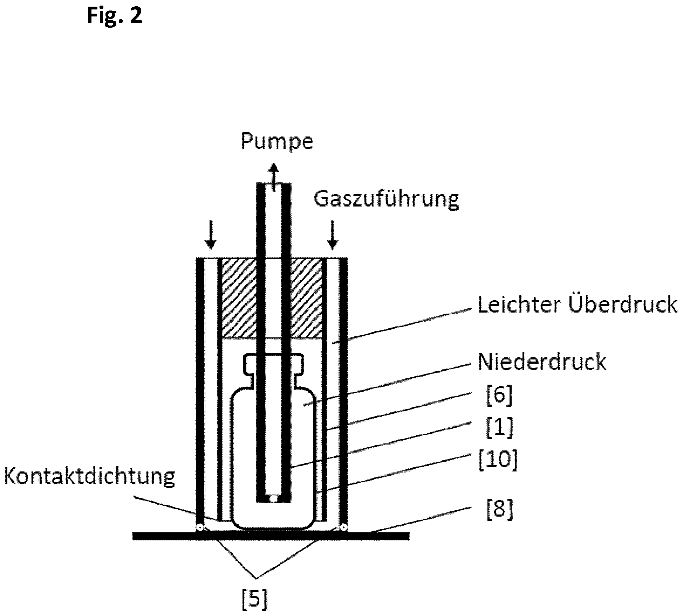

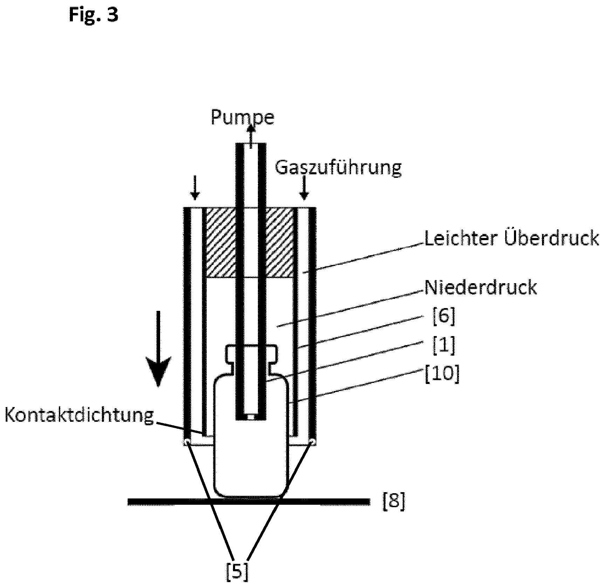

Die Erfindung soll nachfolgend anhand einiger Beispiele näher erläutert werden, ohne die Erfindung auf diese Beispiele zu beschränken. Die Figuren 1, 2, 3, 4 und 5 zeigen die erfindungsgemäße Vorrichtung.

Für die nachfolgenden Zeichnungen werden folgende Bezugszeichen verwendet:
1 elektrisch leitfähiges Innenrohr (-Hochspannungselektrode, Absaugung)
2 Öffnungen im Innenrohr
3 galvanische Isolation durch einen Flansch
4 Außenrohr I
5 Dichtung
6 Außenrohr II (-Erdungselektrode)
7 Kontaktdichtung
8 Grundplatte/Bodenplatte/Transportplatte
9 Öffnung für die Zuführung des Arbeitsgases
10 Behandlungsgut (-Hohlkörper)

Die Vorrichtung gemäß einer bevorzugten Ausführungsform der Erfindung ist in den Figuren 1, 2 und 3 schematisch dargestellt.

Die Vorrichtung verfügt gemäß Figuren 1 bis 3 über ein elektrisch leitfähiges Innenrohr 1, über das Gase ab- oder zugeführt werden können. Hierzu werden Öffnungen im Rohr, z.B. an Position 2, verwendet. Das Innenrohr 1 kann als Innenelektrode einer dielektrisch behinderten Entladung oder als koaxialer Innenleiter einer Hochfrequenz- oder Mikrowellenzuführung dienen. Hierzu kann er auch mit speziellen Strukturen versehen werden, um eine bessere Zündung des Plasmas zu gewährleisten. Das Innenrohr kann zur zusätzlichen Isolation oder zur Erhöhung der chemischen Beständigkeit mit einem Dielektrikum ummantelt sein. Das Innenrohr ist galvanisch isoliert - vorzugsweise durch einen Flansch 3 - mit zwei Außenrohren 4, 6 verbunden. Mindestens eines davon ist elektrisch leitfähig. Die beiden Rohre können sich auf demselben Potential befinden. Besonders vorteilhaft ist es, dieses auf Erdpotential zu setzen. Auch hier können dielektrische Ummantelungen eingesetzt werden. Auch kann das Rohr 6 als weiteres Dielektrikum genutzt werden. Das oder die elektrisch leitenden Außenrohre bilden dann im Falle des Betriebs als dielektrisch behinderte Entladung die zweite Elektrode. Im Fall einer Hochfrequenz- oder Mikrowellenanregung des Plasmas stellt es den koaxialen Außenleiter dar. Ferner kann das für eine dielektrisch behinderte Entladung erforderliche Dielektrikum auch durch das Behandlungsgut selbst dargestellt werden.

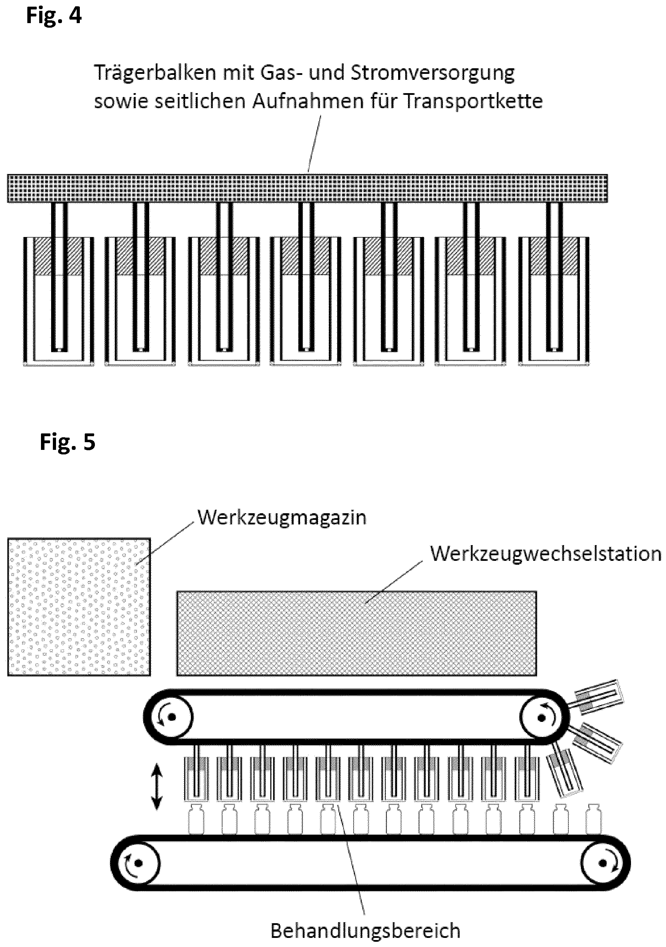

Die Vorrichtung wird zur Behandlung über das Behandlungsgut geschoben (siehe Figuren 4 und 5) und das äußere Außenrohr 4 kontaktiert die vorzugsweise geerdete Grundplatte 8. Durch eine Dichtung in 5 wird eine weitestgehend gasdichte Verbindung hergestellt.

Durch eine Zuführung der Arbeitsgase durch die Öffnungen 9 und Absaugung der Gase durch die Öffnungen 2 im Innenrohr kann bei entsprechendem Spalt-Maß zwischen 6 und 8 bei entsprechendem Glasfluss im Spalt zwischen 4 und 6 ein leichter Überdruck erzielt werden. Wohingegen im Behandlungsbereich zwischen 6 und 1 Normaldruck bis Unterdruck herrschen. Durch diese Betriebsweise wird erreicht, dass die Anforderungen an das Dichtsystem 5, 7 nicht hoch sind. Sollte diese Dichtung versagen, führt dies nicht zu einer Verunreinigung des Prozessgases und einem damit einhergehenden Prozessausfall. Die Konsequenz eines Dichtungsversagens ist primär ein erhöhter Gasverbrauch, der leicht zu detektieren ist und behoben werden kann.

Um eine verbesserte Anpassung der Druckverhältnisse im Zwischenraum zwischen 4 und 6 und den Behandlungsraum zwischen 1 und 6 zu erzielen, kann das Spalt-Maß von 6 zu 8 variiert werden. Es kann z. B. auch in 6 eine zusätzliche Dichtung eingebaut werden und die Gaszuführung über Bohrungen durch die Wandungen erzielt werden. Diese können z.B. reguliert werden, indem 6 zweiteilig ausgeführt wird und die Bohrungen durch verdrehen der beide konzentrischen Rohre gegeneinander geöffnet bzw. verschlossen werden können.

Damit sind Verfahren und Vorrichtung besonders geeignet für eine hohe Anzahl an Lastzyklen.

Zur Spülung, z.B. um sich im Prozess bildende toxische Gase zu entfernen, können der Gas-Strom sowie die Druckbereiche umgekehrt werden.

Zusätzlich kann durch zusätzliche Gaskanäle in 6 bzw. schräge Nuten im Boden von 6 zwischen 6 und dem Behandlungsgut eine tangentiale Gasströmung erzeugt werden, die zu einer Zentrierung des Behandlungsgutes führt. Durch eine Erhöhung der Strömungsgeschwindigkeit kann ein Abheben des Behandlungsgutes von der Bodenplatte erreicht werden und so auch eine Behandlung des äußeren Bodenbereiches des Behandlungsgutes erreicht werden.

Eine weitere Möglichkeit zur Abhebung des Behandlungsgutes von der Bodenplatte 8 kann erreicht werden, wenn das Innenrohr 1 bzw. Teile davon bei einer zweiteiligen Ausführung sich im Verhältnis so zu den Außenrohren 4 und 6 bewegen können, dass über eine entsprechende Vorrichtung der Boden der Flasche angesaugt werden kann und damit dann das Behandlungsprodukt angehoben werden kann, so dass die Unterseite behandelt werden kann.

Auch kann durch diese Methode im Falle von Flaschen und Hohlkörpern eine Dichtwirkung zwischen Behandlungsgut und Flansch 3 erzielt werden, der zu einem Druckgefälle zwischen Innenraum und Außenraum des Behandlungsgutes führt und für die Prozessführung und Plasmaausbildung vorteilhaft genutzt werden kann.

## Patentansprüche

1. Vorrichtung zur Plasmabehandlung von Hohlkörpern (10), umfassend eine Grundplatte (8) und ein elektrisch leitendes Innenrohr (1), **dadurch gekennzeichnet, dass** die Vorrichtung weiterhin zwei Außenrohre (4, 6) aufweist, nämlich ein inneres Außenrohr (6) und ein äußeres Außenrohr (4), von denen mindestens eines elektrisch leitfähig ist, und das Innenrohr (1) und das innere Außenrohr (6)voneinander galvanisch isoliert sind und miteinander gasdicht verbunden sind, so dass sie eine Vorkammer zwischen den zwei Außenrohren (4, 6) und einen Behandlungsraum zwischen dem inneren Außenrohr (6) und dem Innenrohr (1) bilden, wobei das äußere Außenrohr (4) mit der Grundplatte (8) über eine Dichtung (5) verbindbar oder verbunden ist, und das innere Außenrohr (6) über eine einstellbare Kontaktdichtung (7) mit der Grundplatte (8) verbindbar oder verbunden ist.

2. Vorrichtung nach Anspruch 1, **dadurch gekennzeichnet, dass** das Innenrohr (1) mindestens eine Öffnung (2) und die Vorkammer mindestens eine Öffnung (9) zum Einfüllen oder Absaugen von Gasen aufweist.

3. Vorrichtung nach Anspruch 1 oder 2, **dadurch gekennzeichnet, dass** die galvanische Isolierung durch einen Flansch (3) realisiert ist.

4. Vorrichtung nach einem der Ansprüche 1 bis 3, **dadurch gekennzeichnet, dass** sich die beiden Außenrohre (4, 6) und die Grundplatte (8) auf demselben Potential, vorzugsweise auf Erdpotential, befinden.

5. Vorrichtung nach einem der Ansprüche 1 bis 4, **dadurch gekennzeichnet, dass** das Innenrohr (1) und die beiden Außenrohre (4, 6) konzentrisch angeordnet sind.

6. Vorrichtung nach einem der Ansprüche 1 bis 5, **dadurch gekennzeichnet, dass** das Innenrohr im Fall einer dielektrisch behinderten Entladung als Hochspannungselektrode fungiert und mit einem Dielektrikum ummantelt ist.

7. Vorrichtung nach einem der Ansprüche 1 bis 5, **dadurch gekennzeichnet, dass** das innere Außenrohr (6) oder der Hohlkörper (10) als Dielektrikum ausgestaltet ist.

8. Vorrichtung nach einem der Ansprüche 1 bis 7, **dadurch gekennzeichnet, dass** das innere Außenrohr (6) zweiteilig ausgeführt ist und / oder zusätzliche Öffnungen für Gase und / oder eine zusätzliche Dichtung enthält.

9. Vorrichtung nach einem der Ansprüche 1 bis 8, **dadurch gekennzeichnet, dass** das innere Außenrohr (6) zusätzliche Gaskanäle bzw. schräge Nuten im Boden aufweist.

10. Vorrichtung nach einem der Ansprüche 1 bis 9, **dadurch gekennzeichnet, dass** auf der Seite, die der Behandlungsstelle abgewandt ist, eine Werkzeugwechselstation und ein Werkzeugmagazin installiert ist.

11. Balkensystem, welches mehrere Vorrichtungen nach einem der Ansprüche 1 bis 9, aufweist, **dadurch gekennzeichnet, dass** mehrere Einzelvorrichtungen zu Balken kombiniert sind, die durch beidseitige Transportketten parallel mit dem Transport von zu behandelnden Vials mitführbar sind.

12. Verfahren zur Plasmabehandlung von Hohlkörpern mittels der Vorrichtung gemäß einem der Ansprüche 1 bis 10, **dadurch gekennzeichnet, dass** die Plasmabehandlung zwischen dem inneren Außenrohr (6) und dem Innenrohr (1) durchgeführt wird

13. Verfahren zur Plasmabehandlung von Hohlkörpern nach Anspruch 12, **dadurch gekennzeichnet, dass** ein zu behandelnder Hohlkörper (10) als Dielektrikum fungiert.

14. Verwendung der Vorrichtung gemäß einem der Ansprüche 1 bis 10 zur Dekontamination, Desinfektion, Sterilisation, Reinigung, Aktivierung oder Beschichtung von Hohlkörpern, vorzugsweise von Vials, Flaschen, Tuben oder Verschlusskappen.

## Claims

1. An apparatus for plasma treatment of hollow bodies (10), comprising a base plate (8) and an electrically conductive inner tube (1), **characterized in that** the apparatus further has two outer tubes (4, 6), specifically an internal outer tube (6) and an external outer tube (4), at least one of which is electrically conductive, and the inner tube (1) and the internal outer tube (6) are galvanically isolated from one another and are gas-tightly interconnected so as to form a prechamber between the two outer tubes (4, 6) and a treatment chamber between the internal outer tube (6) and the inner tube (1), wherein the external outer tube (4) is connectable or connected to the base plate (8) via a seal (5), and the internal outer tube (6) is connectable or connected to the base plate (8) via an adjustable contact seal (7).

2. The apparatus according to Claim 1, **characterized in that** the inner tube (1) has at least one opening (2), and the prechamber comprises at least one opening (9) for introducing or suctioning gases.

3. The apparatus according to Claim 1 or 2, **characterized in that** the galvanic isolation is achieved by a flange (3).

4. The apparatus according to any one of Claims 1 to 3, **characterized in that** the two outer tubes (4, 6) and the base plate (8) are at the same potential, preferably at earth potential.

5. The apparatus according to any one of Claims 1 to 4, **characterized in that** the inner tube (1) and the two outer tubes (4, 6) are concentrically arranged.

6. The apparatus according to any one of Claims 1 to 5, **characterized in that** the inner tube acts as a high-voltage electrode in the case of a dielectrically impeded discharge and is surrounded by a dielectric.

7. The apparatus according to any one of Claims 1 to 5, **characterized in that** the internal outer tube (6) or the hollow body (10) is designed as a dielectric.

8. The apparatus according to any one of Claims 1 to 7, **characterized in that** the internal outer tube (6) is configured in two parts, and/or contains additional openings for gases and/or an additional seal.

9. The apparatus according to any one of Claims 1 to 8, **characterized in that** the internal outer tube (6) has additional gas channels or angled grooves in the base.

10. The apparatus according to any one of Claims 1 to 9, **characterized in that**, on the side facing away from the treatment location, a tool changing station and a tool magazine are installed.

11. A beam system having a plurality of apparatuses according to any one of Claims 1 to 9, **characterized in that** a plurality of individual apparatuses are combined to form beams, which can be carried along by transporting chains on both sides in parallel with the transportation of vials to be treated.

12. A method for plasma treatment of hollow bodies by means of the apparatus according to any one of Claims 1 to 10, **characterized in that** the plasma treatment is carried out between the internal outer tube (6) and the inner tube (1).

13. The method for plasma treatment of hollow bodies according to Claim 12, **characterized in that** a hollow body (10) to be treated acts as a dielectric.

14. The use of the apparatus according to any one of Claims 1 to 10 for decontaminating, disinfecting, sterilizing, cleaning, activating or coating hollow bodies, preferably vials, bottles, tubes or closure caps.

## Revendications

1. Dispositif de traitement de corps creux (10) au plasma, comprenant une plaque de base (8) et un tube interne (1) électriquement conducteur, **caractérisé en ce que** le dispositif présente encore deux tubes externes (4, 6), à savoir un tube externe intérieur (6) et un tube externe extérieur (4), dont l'un au moins est électriquement conductible, et que le tube interne (1) et le tube externe intérieur (6) sont isolés galvaniquement l'un de l'autre et sont reliés l'un à l'autre de façon étanche aux gaz, de sorte qu'ils forment une préchambre entre les deux tubes externes (4, 6) et un espace de traitement entre le tube externe intérieur (6) et le tube interne (1), dans lequel le tube externe extérieur (4) peut être relié ou est relié à la plaque de base (8) par un joint d'étanchéité (5), et le tube externe intérieur (6) peut être relié ou est relié à la plaque de base (8) au moyen d'un joint d'étanchéité par contact (7).

2. Dispositif selon la revendication 1, **caractérisé en ce que** le tube interne (1) présente au moins une ouverture (2) et la préchambre comporte au moins une ouverture (9) destinée au remplissage ou à l'évacuation de gaz.

3. Dispositif selon la revendication 1 ou 2, **caractérisé en ce que** l'isolation galvanique est réalisée par une bride (3).

4. Dispositif selon l'une quelconque des revendications 1 à 3, **caractérisé en ce que** les deux tubes externes (4, 6) et la plaque de base (8) se trouvent sur le même potentiel, de préférence sur le potentiel de la terre.

5. Dispositif selon l'une quelconque des revendications 1 à 4, **caractérisé en ce que** le tube interne (1) et les deux tubes externes (4, 6) sont disposés de façon concentrique.

6. Dispositif selon l'une quelconque des revendications 1 à 5, **caractérisé en ce que** le tube interne sert d'électrode à haute tension dans le cas d'une décharge à barrière diélectrique et est entouré par un diélectrique .

7. Dispositif selon l'une quelconque des revendications 1 à 5, **caractérisé en ce que** le tube externe intérieur (6) ou bien le corps creux (10) est conçu sous la forme d'un diélectrique.

8. Dispositif selon l'une quelconque des revendications 1 à 7, **caractérisé en ce que** le tube externe intérieur (6) est réalisé en deux parties et / ou comprend des ouvertures supplémentaires destinées aux gaz et / ou un joint d'étanchéité supplémentaire.

9. Dispositif selon l'une quelconque des revendications 1 à 8, **caractérisé en ce que** le tube externe intérieur (6) présente des arrivées de gaz supplémentaires ou des rainures obliques dans la base.

10. Dispositif selon l'une quelconque des revendications 1 à 9, **caractérisé en ce qu'**un changeur d'outils et un magasin d'outils sont installés du côté opposé au poste de traitement.

11. Système à faisceau, lequel présente plusieurs dispositifs selon l'une quelconque des revendications 1 à 9, **caractérisé en ce que** plusieurs dispositifs individuels sont combinés en un faisceau, qui peuvent être entraînés par des chaînes de transport bilatérales parallèlement au transport de flacons à traiter.

12. Procédé de traitement de corps creux au plasma à l'aide du dispositif selon l'une quelconque des revendications 1 à 10, **caractérisé en ce que** le traitement au plasma est exécuté entre le tube externe intérieur (6) et le tube interne (1).

13. Procédé de traitement par plasma de corps creux selon la revendication 12, **caractérisé en ce qu'**un corps creux à traiter (10) sert de diélectrique.

14. Utilisation du dispositif selon l'une quelconque des revendications 1 à 10 à des fins de décontamination, de désinfection, de stérilisation, de nettoyage, d'activation ou de revêtement de corps creux, de préférence de flacons, de bouteilles, de tubes ou de capuchons.
